# EUROPÄISCHE PATENTANMELDUNG

(11) **EP 0 993 802 A1**
(43) Veröffentlichungstag der Anmeldung: **19.04.2000**
(21) Anmeldenummer: 98810936.9
(22) Anmeldetag: 18.09.1998
(51) Int. Cl.: A61B 1/04, A61B 6/14, A61B 1/273

(54) **Verfahren zur Verbesserung der Aufnahmequalität bei videotechnischen Abbildungsverfahren in der Zahnheilkunde, Zubehör für eine zahnärztliche Kamera, Stativsatz**

(71) Anmelder: Mettler, Max, Dr. med. dent., 7522 La Punt-Chamues (CH)
(72) Erfinder: Mettler, Max, Dr. med. dent., 7522 La Punt-Chamues (CH)
(74) Vertreter: Riederer, Conrad A., Dr.

(57) **Zusammenfassung**

Mit einem Stativ (11) für eine zahnmedizinische Vermessungskamera lässt sich die Aufnahmequalität verbessern, während die Zeit für die Positionierung der Optik (13) der Kamera verkürzt und die Übereinstimmung von zwei Aufnahmen aus der gleichen Position erhöht werden kann. Das Stativ (11) sitzt verschiebbar auf dem Objektivgehäuse (13) und wird mit den Abstützungen (27,27') neben dem abzubildenden Bereich z.B. auf den Zähnen abgestellt. Die Abstützungen (27,27') sind mit zahnärztlichen Werkzeugen und Materialien auf die jeweiligen Gegebenheiten anpassbar.

## Beschreibung

### Technisches Gebiet der Erfindung

Die Erfindung betrifft ein Verfahren zur Verbesserung der Aufnahmequalität bei videotechnischen Abbildungsverfahren in der Zahnheilkunde, sowie Zubehör zu einer zahnärztlichen Kamera, insbesondere für intraorale Vermessungsaufnahmen, sowie einen Stativsatz.

### Stand der Technik

Seit einigen Jahren werden Vermessungskameras in der Zahnheilkunde eingesetzt. Mit ihnen werden Videoaufnahmen von einzelnen Zähnen oder Zahngruppen gemacht, um über diese Aufnahmen mit einem Rechner den Zahn zu vermessen und in der Regel auch eine Rekonstruktion des Zahnes zu berechnen, welche dann über eine integrierte Schleifeinheit aus einem keramischen Material gefräst wird. Diese automatisch und hochpräzise gefrästen Inlays, Onlays, Verblendschalen, Voll- oder Teilkronen werden dann allenfalls überarbeitet und mit einem speziellen Kunststoff praktisch unsichtbar in, an oder auf den Restzahn geklebt. Die Vorteile dieser automatisierten Vollkeramik-Rekonstruktionen liegen in den Materialeigenschaften des keramischen Materials, der Zeiteinsparung zum Erstellen des Keramikkörpers, der hohen Passgenauigkeit zwischen präpariertem Zahn und Keramikkörper. Zudem werden Zahnabdrücke und Provisorien umgangen, wie auch der Produktionsablauf sehr verkürzt. Eine Schwierigkeit bei der Zahnvermessung ist die Aufnahmezeit, in welcher die Optik der Kamera ruhig gehalten werden muss. Als Aufnahmedauer ist bei dem auf dem Markt führenden Gerät von Siemens, dem Cerec 2, mit 133/1000 Sekunden, also ca. 1/7 Sekunde angegeben. Der Zahnarzt kann die Kamera während dieser Aufnahmedauer nur dann ruhig halten, wenn er wie empfohlen die Optik mit zwei Händen an den Zähnen oder dem Kiefer abstützt. Das bimanuelle Abstützen ist bei einigen Lokalisationen eines abzubildenden und zu berechnenden Zahns jedoch gar nicht möglich.

Für die Reproduktion einer bestehenden Kaufläche kann diese vor der Präparation des Zahns mit der Kamera vermessen werden. Nach der Präparation benötigt das den Keramikeinsatz berechnende Programm eine zweite Aufnahme. Aus beiden Aufnahmen zusammen wird dann der Keramikeinsatz berechnet, so dass er einerseits exakt in die Präparation passt und andererseits die übernommene Oberflächenform aufweist. Diese beiden Aufnahmen müssen deckungsgleich sein, damit der Rechner die beiden Messungen richtig zusammenfügen, bzw. korrelieren kann, und damit der Keramikkörper anschliessend entsprechend richtig ausgefräst wird. Nun benötigt ein Zahnarzt aber grosses Geschick, die Objektivposition bei der zweiten Aufnahme exakt in Übereinstimmung mit der Objektivposition bei der ersten Aufnahme zu bringen. Eine Kontrolle der Positionierung geschieht durch die Überblendung der beiden Aufnahmen auf dem Bildschirm, wobei zwei relevante Konturen der ersten Aufnahme mit der zweiten Aufnahme zur Deckung zu bringen sind. Leicht kann jedoch der Abstand zwischen Objektiv und Zahn oder der Winkel in irgendeiner Richtung nicht exakt stimmen, und schon stimmen die Aufnahmen nicht überein. Wie nun die Position der Optik zu korrigieren ist, wenn die beiden Bilder in einer oder der anderen Weise nicht übereinstimmen, braucht einige Erfahrung. In der gewünschten Position dann noch die Aufnahme nicht zu verwackeln, ist eine zusätzliche Herausforderung.

### Aufgabe der Erfindung

Aufgabe der Erfindung ist, die Qualität der Vermessungsaufnahme zu erhöhen und die Erstellung von zwei deckungsgleichen Aufnahmen zu vereinfachen.

### Beschreibung der Erfindung

Zur Erhöhung der Aufnahmequalität und Vereinfachung der Erstellung von zwei deckungsgleichen Aufnahmen bei videotechnischen Abbildungsverfahren in der Zahnheilkunde wird die Optik der Kamera gegenüber dem den zu vermessenden oder schlicht abzubildenden Teil tragenden Kiefer erfindungsgemäss unter Zuhilfenahme eines Stativs abgestützt. Dank dem Stativ kann die Optik der Kamera einhändig gehalten werden. Das Stativ verringert die Gefahr des Verwackelns während der Aufnahmedauer. Das Stativ hat weiter den Vorteil, dass es eine konstante Position gegenüber der Optik hat, so dass bei wiederholter gleicher Positionierung des Stativs sich auch die Optik wiederholt an der gleichen Stelle befindet. Ein Stativ kann auch auf die speziellen Gegebenheiten im Mund des Patienten angepasst werden, so dass es einen sehr guten Halt darin findet.

Wenn auch vorstellbar ist, dass das Stativ im Behandlungsraum aufgestellt und der abzubildende Teil gegenüber dem Stativ ruhiggestellt wird, so wird vorteilhaft das Stativ lediglich neben dem abzubildenden Teil, z.B. auf einem Zahn, einem Protheseteil oder auf dem Zahnfleisch, abgestellt. Dadurch kann es jede Bewegung des Kiefers mitmachen, ohne dass dabei die Stellung der Optik gegenüber dem abzubildenden Teil verändert würde.

Vorteilhaft wird die Optik zur präzisen Bildausschnittwahl im Stativ verschoben. Die Verschiebbarkeit der Optik im Stativ ermöglicht die optimale Wahl der Abstützpunkte im Mund und gleichzeitig eine optimale Wählbarkeit des Bildausschnitts. Die Abstützpunkte müssen daher nicht in einem bestimmten Abstand zum abzubildenden Teil gefunden werden.

Dank je nach Anwendung und anatomischer Gegebenheit wählbarer unterschiedlicher Abstützung des Stativs, z.B. einseitig oder beidseitig des abzubildenden Zahns, mittels härtendem, plastischem Material, oder vorgegebenen oder individuell modellierten Abstützbalken etc., kann auf die individuellen Gegebenheiten im Mund des Patienten und auf die Vorlieben des Arztes eingegangen werden.

Die Erfindung schlägt als Zubehör für eine zahnmedizinischen Kamera ein Stativ für deren Optik vor, um damit die Optik am Kiefer abzustützen. Dieses Stativ weist vorteilhaft einen Halterteil zum Aufsetzen auf die Optik einer Kamera auf, und einen Stützteil, um das Stativ neben dem abzubildenden Zahn, z.B. auf einen Zahn oder auf das Zahnfleisch, aufzulegen. Dies erhöht die erreichte Qualität der Aufnahmen, da die Optik gegenüber dem abzubildenden Teil ruhiger gehalten werden kann, als wenn sie lediglich von Hand abgestützt ist. Das Stativ erleichtert die Feinausrichtung der Kamera unter Kontrolle des Bildausschnittes auf dem Bildschirm und erleichtert dadurch das erstellen einer deckungsgleichen Vermessungsaufnahme. Halterteil und Stützteil gewährleisten die konstante Beziehung zwischen Objekt und Objektiv während der gesamten Aufnahmedauer.

Vorteilhaft ist das Halterteil ein Ring oder eine Spange zur Aufnahme der Optik, um ein einfaches Aufsetzen auf und Entfernen des Stativs von einer herkömmlichen Optik zu ermöglichen. Der Ring oder die Spange umschliesst den Umriss des Objektivgehäuses. Das Gehäuse ist in der Spange oder dem Ring verschiebbar und daraus herausziehbar.

Vorteilhaft weist das Stativ einen Rahmen auf, innerhalb welchen das optische Fenster eines mit dem Stativ zusammengefügten Objektivs fällt. Dieser Rahmen ist in wenigstens einer Dimension, nämlich in Richtung der Verschiebbarkeit des Stativs gegenüber dem Objektiv, grösser als die Aussenmasse des optischen Fensters, so dass das optische Fenster des Objektivs innerhalb des Rahmens verschoben werden kann, ohne dass der Bildausschnitt durch den Rahmen beschnitten wird. Der Rahmen verbindet dabei eine vordere und hintere Stützpartie, bzw. eine vordere Stützpartie mit dem Halterteil des Stativs.

Vorteilhaft besteht das Stativ aus einem durch den Anwender bearbeitbaren Material. Dies lässt dem Zahnarzt freie Hand, die Stützbereiche des Stativs an die jeweiligen Gegebenheiten anzupassen.

Zweckmässigerweise ist das Stativ aus einem wiederholt im Autoklav sterilisierbaren, Desinfektionslösungen verträglichen und UV-resistenten Kunststoff hergestellt, damit es den hygienischen Anforderungen in einer Zahnarztpraxis entsprechend behandelt und mehrfach verwendet werden kann.

Die Erfindung betrifft auch einen Stativsatz mit einer Mehrzahl von solchen Stativen mit unterschiedlichen Ausformungen der Abstützungen, z.B. mit lediglich vorne einer Abstützung, vorne und hinten je einer gleichen oder unterschiedlichen Abstützung, lediglich hinten einer Abstützung, einer Abstützung in Form eines Stützbalken, eines Stützbeins, oder mit einer Ausnehmung zur Aufnahme eines plastischen, härtenden Materials als Abstützung. Ein solcher Stativsatz liefert dem Zahnarzt unterschiedliche Stative für unterschiedliche Anforderungen.

Weiter betrifft die Erfindung auch einen Stativsatz mit einer Mehrzahl von Stativen zur individuellen Bearbeitung der Abstützungen durch den Anwender. Es können sowohl unterschiedliche Stative darin vorliegen. Eine Mehrzahl von identischen Stativen wird vorgezogen.

### Kurzbeschrieb der Figuren

Es zeigt:
- Fig. 1: eine perspektivische Darstellung eines Stativs,
- Fig. 2: eine perspektivische Darstellung desselben Stativs, jedoch auf ein Objektiv aufgesetzt,
- Fig. 3: ein Objektiv mit aufgesetztem Stativ in Aufnahmeposition im Backenzahnbereich,
- Fig. 4: ein Objektiv mit aufgesetztem Stativ in Aufnahmeposition im Frontzahnbereich,
- Fig. 5: ein Stativ mit lediglich einer vorderen Abstützung,
- Fig. 6: ein Stativ mit einer keilförmigen hinteren Abstützung,
- Fig. 7: ein Stativ mit aus plastischem, härtendem Material erstellter hinterer Abstützung,
- Fig. 8: ein Stativ mit individuell bearbeiteten Abstützbalken.

### Beschrieb der Ausführungsbeispiele

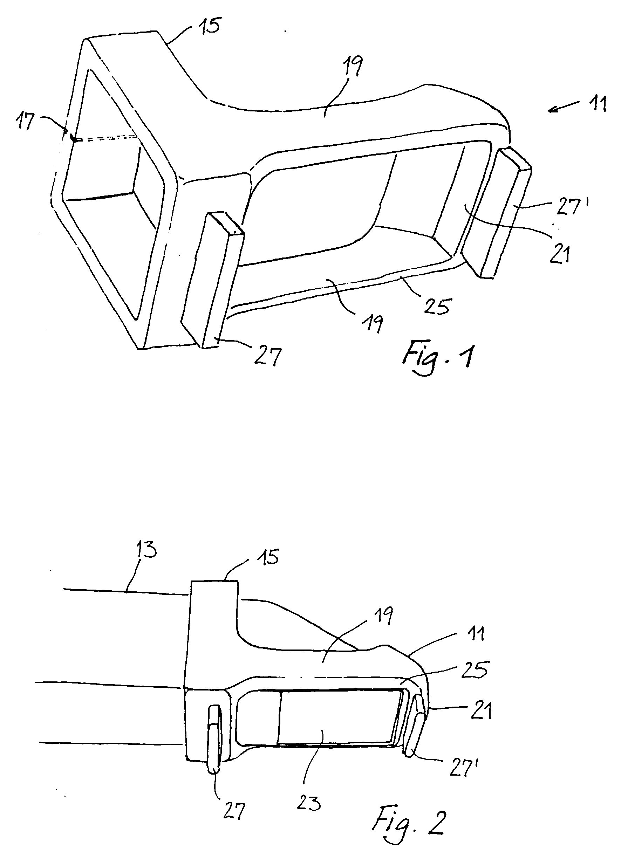

Das in den Figuren 1 und 2 dargestellte Stativ 11 ist ein auf ein Objektivgehäuse 13 einer Vermessungskamera aufsteckbares und vom Objektivgehäuse 13 wieder lösbares Zubehörteil. Das längliche Objektivgehäuse 13 weist an seinem vorderen Ende ein seitlich angeordnetes Objektivfenster 23 auf, durch welches hindurch die Videoaufnahmen gemacht werden. Mit einem Ring 15 sitzt das aufgesteckte Stativ 11 hinter dem Objektivfenster 23 auf dem Objektivgehäuse 13 und ist darauf verschiebbar. Der Ring 15 könnte auch an einer Stelle einen Unterbruch 17 (in Fig. 1 gestrichelt dargestellt) aufweisen. Mit dem Ring 15 oder der den Unterbruch 17 aufweisenden Spange klemmt das Stativ 11 am Objektivgehäuse 13 fest.

Vom Halterteil, d.h. dem Ring 15 oder allenfalls der Spange aus erstrecken sich zwei Arme 19 parallel gegen ein diese Arme verbindendes Verbindungsteil 21, welches in einem Abstand zum Ring 15 angeordnet ist. In aufgesetztem Zustand begleiten die Arme 19 das Objektivgehäuse 13 beidseitig des Objektivfensters 23. Der Ring 15, die Arme 19 und das Verbindungsteil 21 bilden zusammen einen Rahmen 25, welcher in auf das Objektiv 13 aufgesetztem Zustand das Objektivfenster 23 umrahmen. Das Öffnungsmass des Rahmens 25 ist zwischen Ring 15 und Verbindungsteil 21 grösser als die entsprechende Länge des Objektivfensters 23. Durch die Klemmverbindung zwischen Ring 15 und Objektivgehäuse 13 ist das Stativ 11 auf dem Objektiv 13 in Richtung der Arme 19 verschiebbar, in welcher Richtung das Öffnungsmass des Rahmens 25 wesentlich länger ist als das Objektivfenster 23. Ein zweckmässiger Verschiebungsspielraum liegt zwischen 5 und 10 mm.

Am Ring 15 und am Verbindungsteil 21 ist je ein Stützbalken 27,27' angeordnet. Ring 15, Arme 19, Verbindungsteil 21 und Stützbalken 27,27' sind zusammen einstückig aus einem Kunststoff hergestellt. Der Kunststoff ist durch den Zahnarzt, Zahntechniker oder Laborant mit seinen gewöhnlichen Werkzeugen bearbeitbar. Er ist auch sterilisierbar und durch UV-Bestrahlung entkeimbar, ohne dass dadurch die Materialeigenschaften rasch beeinträchtigt würden.

Die Stützbalken 27,27' sind in Figur 1 und 2 in einer Grundform dargestellt, welche verschiedene Abwandlungen erlaubt. Diese Abwandlungen können als Fertigteile vorliegen oder durch den Benutzer des Stativs 11 durch Abänderung der Grundform individuell hergestellt werden. Die Grundform der Stützbalken 27,27' ist etwa 3 bis 4 mm hoch, 12 bis 20 mm lang und 2 bis 3 mm breit.

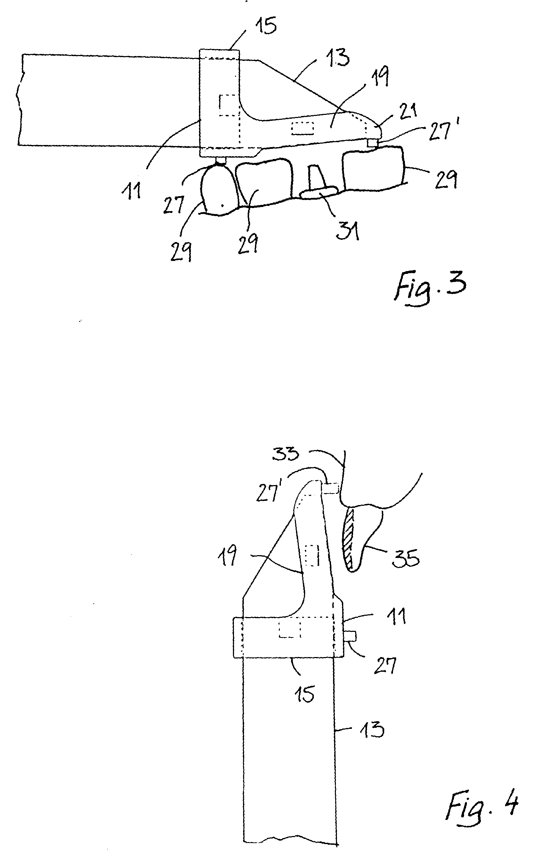

Für eine Anwendung im Backenbereich, wie in Figur 3 dargestellt, werden die Stützbalken 27,27' unter Umständen zweckmässigerweise bis auf etwa 1.5 mm abgetragen. Dies kann auch lediglich in einem auf einem Zahn 29 aufliegenden Teilbereich des Stützbalkens 27,27' geschehen. Dadurch liegen auch die tiefstliegenden Bereiche des abzubildenden und zu vermessenden Teils 31 - in Figur 3 ist dies ein Stift für eine Krone - noch im Schärfebereich der Kamera.

Für eine Anwendung im Frontzahnbereich, wie es in Figur 4 dargestellt ist, wird der Stützbalken 27' vorteilhaft in seiner ganzen Höhe und Länge stehengelassen und auf dem Zahnfleisch 33 oberhalb (bzw. bei unteren Frontzähnen unterhalb) des zu vermessenden Zahns 35 abgestellt. Der hintere Stützbalken 27 wird dabei nicht benötigt.

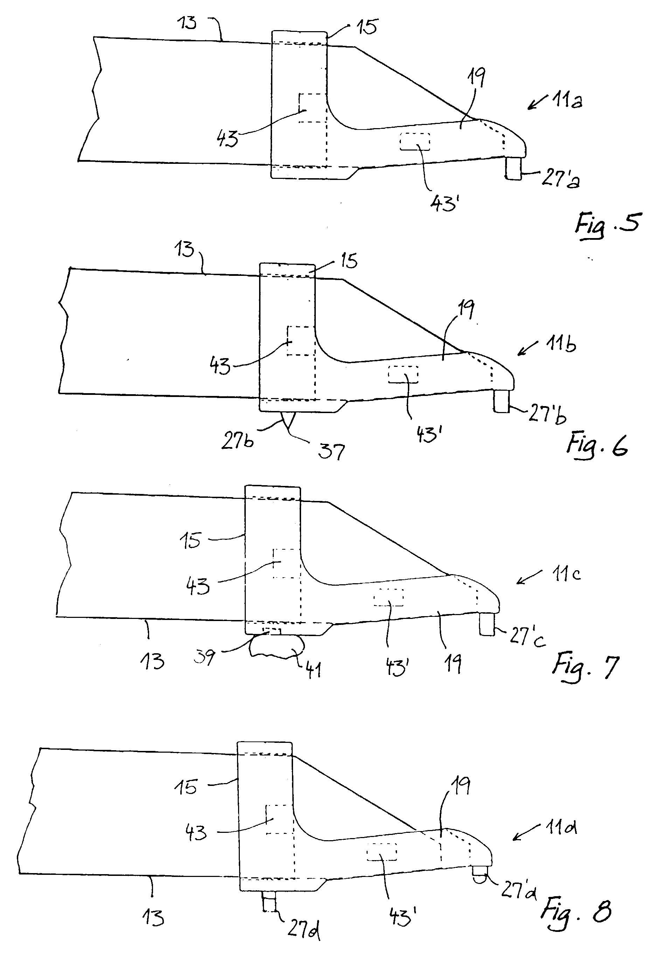

In den Figuren 5 bis 8 sind verschiedene Ausformungen der Abstützungen 27,27' dargestellt. In Figur 5 fehlt die hintere Abstützung 27. Dieses Stativ 11a ist daher für den Frontzahnbereich geeignet. In Figur 6 ist am Stativ 11b die hintere Abstützung 27b keilförmig zugespitzt. Mit der Keilspitze 37 kann im Zwischenraum zwischen zwei Zähnen eingerastet werden, während der vordere, hier in der Grundform vorliegende Stützbalken 27'b auf einen Zahn aufgelegt werden kann.

In Figur 7 ist ein wichtiges Ausführungsbeispiel 11c des Stativs dargestellt. An der Stelle des hinteren Stützbalkens 27 sind Retentionen 39 im Rahmen 15 angeordnet. In diese Retentionen 39 kann eine thermoplastische oder chemisch härtende, plastische Masse 41 gedrückt werden. Mit dieser Masse 41 aufgebracht wird dann das Stativ 11c in Aufnahmeposition gebracht. Dabei passt sich die Masse 41 der Zahnoberfläche, auf die sie gepresst wird, an, und härtet aus. Die ausgehärtete Masse 41 kann nun für eine zweite Aufnahme vom gleichen Standort aus als exakte Führung und Abstützung des Stativs dienen. Der Abdruck der Zahnoberfläche kann ohne Mühe an die genau gleiche Stelle gesetzt werden. Dabei lässt die Elastizität der ausgehärteten Masse 41 eine leichte Korrektur der Position durchaus zu. Eine solche Masse kann vorne oder hinten angebracht werden, oder auch vorne und hinten. Als härtende, plastische Masse 41 können die für Gebissabdrücke gebräuchlichen Materialien, wie Silikone, Wachse, Kunststoffe verwendet werden.

In Figur 8 ist ein Stativ 11d dargestellt, mit an die speziellen Gegebenheiten eines Patienten angepassten Stützbalken 27,27'. Dieses Ausführungsbeispiel 11d soll verdeutlichen, dass die Stützbalken auch in Querrichtung individuell den Bedürfnissen angepasst werden können, und dies während der Sitzung, im Laufe der Behandlung des Patienten. Weiter zeigt die Figur 8, dass durch Verschieben des Objektivs 13 im Stativ 11d der Bildauschnitt gewählt werden kann. Das Objektiv 13 ist in Figur 8 - gegenüber zum Objektiv in Figur 7 z.B. - zum Halterteil hin, d.h. zum Ring 15 hin verschoben.

Da die heute gebräuchlichen Objektivgehäuse 13 leicht konisch nach vorne zusammenlaufend ausgestaltet sind, muss das Halterteil 15 auf verschiedenen Querschnitten des Objektivgehäuses 13 klemmen. Dazu ist auf der Innenseite des Rings 15 etwa mittig auf jeder der vier Seiten des rechteckigen Querschnitts eine leicht vorstehende Friktionsfläche 43 ausgebildet. Dadurch besteht in den Eckbereichen ein kleiner Zwischenraum zwischen dem Ring 15 und dem Objektiv 13. Bei grösserem Querschnitt des Objektivs sind die Friktionsflächen 43 stärker auseinandergedrückt und die Eckpartien des Rings 15 dem Objektivgehäuse 13 stärker angenähert. Ein minimaler Zwischenraum zwischen Gehäuse 13 und Ring 15 ermöglicht diese leichte Verformung des Rings 15. Je eine weitere Friktionsfläche 43' ist an den Armen 19 ausgebildet, um diese und damit die vordere Abstützung 27' zu stabilisieren.

Zusammenfassend kann gesagt werden, dass sich mit einem Stativ für eine zahnmedizinische Vermessungskamera die Aufnahmequalität verbessern lässt, während die Zeit für die Positionierung verkürzt und die Übereinstimmung von zwei Aufnahmen aus der gleichen Position erhöht werden kann. Das Stativ sitzt verschiebbar auf dem Objektivgehäuse und wird neben dem zu vermessenden Bereich z.B. auf den Zähnen abgestellt. Die Abstützungen sind mit zahnärztlichen Werkzeugen und Materialien auf die jeweiligen Gegebenheiten und Bedürfnisse anpassbar.

## Patentansprüche

1. Verfahren zur Erhöhung der Aufnahmequalität bei videotechnischen Abbildungsverfahren in der Zahnheilkunde, bei welchem die Optik (13) der Kamera gegenüber dem Kiefer (29,33) mit dem abzubildenden Teil (31,35) abgestützt wird, dadurch gekennzeichnet, dass die Abstützung unter Zuhilfenahme eines Stativs (11) geschieht.

2. Verfahren nach Anspruch 1, dadurch gekennzeichnet, dass das Stativ (11) neben dem abzubildenden Teil (31,35), z.B. auf einem Zahn (29) oder auf dem Zahnfleisch (33), abgestellt wird.

3. Verfahren nach Anspruch 1 oder 2, dadurch gekennzeichnet, dass die Optik (13) zur präzisen Bildausschnittwahl im Stativ (11) verschoben wird.

4. Verfahren nach einem der Ansprüche 1 bis 3, dadurch gekennzeichnet, dass je nach Anwendung und anatomischer Gegebenheit eine unterschiedliche Abstützung (27,27'; 27a-d,27'a-d) des Stativs (11,11a-d), insbesondere einseitig oder beidseitig des abzubildenden Teils (31,35) z.B. mittels härtendem, plastischem Material (41), vorgegebenen oder individuell modellierten Abstützbalken (27,27'; 27a-d,27'a-d) etc., gewählt werden kann.

5. Stativ (11) für die Optik (13) einer zahnmedizinischen Kamera, insbesondere einer Vermessungskamera, zur Abstützung der Optik (13) am Kiefer (29,33).

6. Stativ nach Anspruch 5, gekennzeichnet durch einen Halterteil (15) zum Aufsetzen auf die Optik (13) einer Kamera, und einen Stützteil (27,27'; 27a-d,27'a-d), um das Stativ (11) neben dem abzubildenden Teil (31,35), z.B. auf einen Zahn (29) oder auf das Zahnfleisch (33), aufzulegen.

7. Stativ nach Anspruch 6, dadurch gekennzeichnet, dass der Halterteil ein Ring (15) oder ein Spange zur Aufnahme der Optik (13) ist.

8. Stativ nach einem der Ansprüche 5 bis 7, gekennzeichnet durch einen Rahmen (25), innerhalb welchen das optische Fenster (23) eines mit dem Stativ (11) zusammengefügten Objektivs (13) fällt, welcher Rahmen (25) in wenigstens einer Dimension grösser ist als die Aussenmasse des optischen Fensters (23), so dass das optische Fenster (23) des Objektivs (13) innerhalb des Rahmens (25) verschoben werden kann.

9. Stativ nach einem der Ansprüche 5 bis 8, dadurch gekennzeichnet, dass es aus einem durch den Anwender bearbeitbaren Material besteht.

10. Stativsatz mit einer Mehrzahl von Stativen (11,11a-d) gemäss einem der Ansprüche 5 bis 9 mit unterschiedlichen Ausformungen der Abstützungen (27,27'; 27a-d,27'a-d), z.B. mit lediglich vorne einer Abstützung (27'a), vorne und hinten je einer gleichen (27,27') oder unterschiedlichen Abstützung (27b-d,27'b-d), lediglich hinten einer Abstützung, einer Abstützung in Form eines Stützbalken (27,27'), eines Stützbeins, und/oder mit Retentionen (39) zur Aufnahme eines plastischen, härtenden Materials (41) als Abstützung.

11. Stativsatz mit einer Mehrzahl von Stativen (11,11a-d) gemäss einem der Ansprüche 5 bis 9 zur individuellen Bearbeitung der Abstützungen (27,27',27a-d,27'a-d) durch den Anwender.
